(19) Europäisches Patentamt / European Patent Office / Office européen des brevets

(11) **EP 4 745 574 A1**

(12) **EUROPEAN PATENT APPLICATION**
published in accordance with Art. 153(4) EPC

(43) Date of publication:
**20.05.2026 Bulletin 2026/21**

(21) Application number: **24842278.4**

(22) Date of filing: **11.07.2024**

(51) International Patent Classification (IPC):
*G01N 33/564* (2006.01)    *G01N 33/543* (2006.01)
*G01N 33/68* (2006.01)    *G01N 33/58* (2006.01)

(86) International application number:
**PCT/CN2024/104996**

(87) International publication number:
**WO 2025/016284 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023   CN 202310870599**

(71) Applicant: **Shenzhen Ruimeng Innovation Biotechnology Co., Ltd.**
**Shenzhen, Guangdong 518071 (CN)**

(72) Inventors:
• **LIN, Chaoxin**
  **Shenzhen, Guangdong 518071 (CN)**
• **CHEN, Tangming**
  **Shenzhen, Guangdong 518071 (CN)**

(74) Representative: **V.O.**
**P.O. Box 87930**
**2508 DH Den Haag (NL)**

Remarks:
The complete document including Reference Table(s) and the Sequence Listing(s) can be downloaded from the EPO website

(54) **FLUORESCENT IMMUNOCHROMATOGRAPHIC TEST STRIP FOR COMBINED DETECTION OF ANTI-MCV ANTIBODY, ANTI-CCP ANTIBODY AND RF**

(57)    The present application relates to a fluorescent immunochromatographic test strip for detecting rheumatoid arthritis. The test strip comprises a sample loading zone, a detection zone and an absorbent zone, wherein the detection zone comprises a first sub-detection zone coated with mutant citrullinated vimentin, a second sub-detection zone coated with cyclic citrullinated peptide, and a third sub-detection zone coated with denatured human IgG.

Fig. 1

EP 4 745 574 A1

**Description**

**CROSS REFERENCE TO RELATED APPLICATIONs**

The present application claims priority to and the benefit of Chinese Application No. 202310870599.3, filed on July 14, 2023, which is incorporated herein in its entirety for all purposes.

**TECHNICAL FIELD**

The present disclosure relates to the field of medical diagnostics, and in particular to a fluorescent immunochromatographic test strip for simultaneously detecting anti-mutant citrullinated vimentin antibody (Anti-MCV), anti-cyclic citrullinated peptide antibody (Anti-CCP), and rheumatoid factor (RF) and application thereof.

**BACKGROUND**

Rheumatoid arthritis (RA) is a chronic, systemic autoimmune disease. In addition to arthritis and involvement of surrounding tissues, subcutaneous nodules, anemia, pulmonary interstitial lesions, and vasculitis may also be manifest as extra-articular manifestations. Untreated RA can lead to significant joint damage and progress to limb disability and loss of function, placing a significant economic burden on the patient's family and society. With the deepening understanding of the pathogenesis of RA and the development of a numerous high-quality clinical trials, the treatment strategies of RA are constantly updated, and the levels of diagnosis and treatment have improved rapidly. Autoantibodies can be produced before RA patients exhibit clinically typical symptoms and possess certain predictive value, and thus have significant applications in early diagnosis, therapeutic monitoring, and prognostic evaluation of RA. Among them, rheumatoid factor (RF) and anti-citrullinated peptide/protein antibody (ACPA) have been included in 2010 American College of Rheumatology (ACR), European Alliance of Associations for Rheumatology (EULAR), and China RA classification criterias due to their good diagnostic performance. They are widely used in clinical practice and have become one of the most common autoantibody detection tests in clinical laboratories. Other antibodies provide complementary diagnostic value for RA and can be used as combined diagnostic indicator for clinical practice.

RF is the earliest serological indicator used for the diagnosis of RA and is currently the most commonly employed indicator in the clinical practice, with high sensitivity but low specificity, and a certain positive rate in other autoimmune diseases, tumors, infected patients and even normal individuals. ACPA refers to a class of autoantibodies that target citrullinated proteins, including anti-perinuclear factor (APF), anti-keratin antibody (AKA), anti-cyclic citrullinated peptide antibody (Anti-CCP), and anti-mutant citrullinated vimentin antibody (Anti-MCV). Although APF and AKA have high specificity, they require immunofluorescence detection, and the results are susceptible to individual subjectivity, with relatively low sensitivity. Anti-CCP and Anti-MCV both have high specificity and sensitivity. It has been reported that Anti-MCV can be detected in sera of patients with Anti-CCP negative RA, suggesting that Anti-MCV as slightly higher specificity than Anti-CCP, but is less sensitive than Anti-CCP. The sensitivity of the three indicators (RF, Anti-CCP, and Anti-MCV) in diagnosing RA follows the order of Anti-CCP > RF > Anti-MCV, while the specificity follows the order of Anti-MCV > Anti-CCP > RF

The commonly used detection methods for RF are chemiluminescence immunoassay, enzyme linked immunosorbent assay, or immunoturbidimetric. Anti-CCP is currently mainly detected by chemiluminescence immunoassay and enzyme linked immunosorbent assay. At present, while Anti-MCV is primarily detected by enzyme linked immunosorbent assay. These methods have the characteristics of being rapid, automated, quantitative, and having low coefficients of variation. However, they are relatively complex to operate and time-consuming, which makes them less suitable for rapid and convenient testing.

Immunochromatography is a rapid diagnostic technique with advantages in accuracy, speed, and ease of operation. Commonly used labeling materials include colloidal gold, fluorescent materials, and the like. Colloidal gold is primarily detected by macroscopic observation of its color, but for some samples with extremely low antigen or antibody content, the colloidal gold appears very faint, making it difficult to judge the result by visual inspection, low in sensitivity. Also, this method has low sensitivity and cannot achieve highly sensitive quantitative detection. Fluorescent materials have high fluorescence intensity, enabling high-sensitivity quantitative detection.

**SUMMARY OF THE INVENTION**

One of the objectives of the present application is to provide a fluorescent immunochromatographic test strip for the simultaneous detection of anti-mutant citrullinated vimentin antibody (Anti-MCV), anti-cyclic citrullinated peptide antibody (Anti-CCP), and rheumatoid factor (RF), and to provide a kit with high sensitivity, good specificity, strong stability, and ease of use for clinical diagnosis and analysis, which can greatly improve detection and diagnosis efficiency, thereby addressing the issues in the prior art.

In the present application, citrulline mutation site of MCV is selected differently from that of CCP, allowing for a clear distinction between the anti-CCP antibody and the anti-MCV antibody, without any cross-reaction, thereby solving the interference problem between CCP and MCV.

A first aspect of the present application provides a fluorescent immunochromatographic test strip for detecting rheumatoid arthritis, the test strip includes a sample loading zone, a detection zone, and an absorbent zone; the detection zone includes a first sub-detection zone coated with mutant citrullinated vimentin (MCV), a second sub-detection zone coated with cyclic citrullinated peptide (CCP), and a third sub-detection zone coated with denatured human IgG; wherein the coating amount of the MCV, the CCP, or the denatured human IgG is independently 0.15-0.75 μg; the amino acid sequence of the MCV is set forth in SEQ ID NO: 1; the amino acid sequence of the CCP is set forth in SEQ ID NO: 2; the sample loading zone contains fluorescent material-labeled goat anti-human IgG, fluorescent material-labeled denatured human IgG, and fluorescent material-labeled goat anti-rabbit IgG, in a mass ratio of 20:10:1.

In some embodiments, the fluorescent immunochromatographic test strip further comprises a substrate.

In some embodiments, the sample loading zone is divided into a sample zone for receiving the sample, and a labeling zone having fluorescent material-labeled goat anti-human IgG, fluorescent material-labeled denatured human IgG, and fluorescent material-labeled goat anti-rabbit IgG coated thereon.

In some embodiments, the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a quality control zone, which is located downstream of the first detection zone, the second detection zone, or the third detection zone in the flow direction of the sample on the test strip.

In some embodiments, the quality control zone is coated with rabbit IgG, with a coating amount of 0.15-0.75 μg.

In some embodiments, the sample loading zone, the detection zone, and the absorbent zone are arranged sequentially from upstream to downstream, according to the flow direction of the sample on the test strip.

In some embodiments, the coating concentration of the MCV, the CCP, and the denatured human IgG is 0.5-1.5 mg/mL.

A second aspect of the present application provides a kit comprising the fluorescent immunochromatographic test strip of the first aspect and instructions.

A third aspect of the present application provides the use of the fluorescent immunochromatographic test strip of the first aspect in the preparation of a kit for detecting rheumatoid arthritis.

A fourth aspect of the present application provides a method for assessing whether a subject suffers from rheumatoid arthritis or determining the severity of rheumatoid arthritis in a subject, comprising the following steps:

> a. adding a blood sample from the subject to the sample loading zone of the fluorescent immunochromatographic test strip;
>
> the fluorescent immunochromatographic test strip includes a sample loading zone, a detection zone, and an absorbent zone;
> detection zone includes a first sub-detection zone coated with MCV, a second sub-detection zone coated with CCP, and a third sub-detection zone coated with denatured human IgG;
> wherein the coating amount of the MCV, the CCP, or the denatured human IgG is independently 0.15-0.75 μg;
> the amino acid sequence of the MCV is set forth in SEQ ID NO: 1;
> the amino acid sequence of the CCP is set forth in SEQ ID NO: 2;
> the sample loading zone contains a fluorescent material-labeled goat anti-human IgG, fluorescent material-labeled denatured human IgG, and fluorescent material-labeled goat anti-rabbit IgG, in a mass ratio of 20:10:1; and
>
> b. determining qualitative or quantitative results generated from the fluorescent materials in the first sub-detection zone, the second sub-detection zone, and the third sub-detection zone, and assessing whether the subject suffers from rheumatoid arthritis or determining the severity of rheumatoid arthritis based on the qualitative or quantitative results.

In some embodiments, in step (b), when the first sub-detection zone, the second sub-detection zone, and the third sub-detection zone show positive fluorescence results, the subject is diagnosed with rheumatoid arthritis.

In some embodiments, the blood sample is a whole blood sample, a plasma sample, or a serum sample.

In some embodiments, the fluorescent immunochromatographic test strip further comprises a substrate.

In some embodiments, the sample loading zone is divided into a sample zone for receiving the sample, and a labeling zone having fluorescent material-labeled goat anti-human IgG, fluorescent material-labeled denatured human IgG, and fluorescent material-labeled goat anti-rabbit IgG coated thereon.

In some embodiments, the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a quality control zone, which is located downstream of the first sub-detection zone, the second sub-detection zone, or the third sub-detection zone in the flow direction of the sample on the test strip.

In some embodiments, the quality control zone is coated with rabbit IgG, with a coating amount of 0.15-0.75 μg.

In some embodiments, the sample loading zone, the detection zone, and the absorbent zone are arranged sequentially from upstream to downstream, according to the flow direction of the sample on the test strip.

In some embodiments, the coating concentration of the MCV, the CCP, and the denatured human IgG is 0.5-1.5 mg/mL.

## BRIEF DESCRIPTION OF THE DRAWINGS

FIG. 1 shows a schematic diagram of a fluorescent immunochromatographic test strip. Where 1 is the sample loading zone, 2 is the detection zone, 3 to 5 are sub-detection zones, 6 is the quality control zone, 7 is the absorbent zone, and 8 is the substrate.

FIG. 2 shows a schematic diagram of another fluorescent immunochromatographic test strip. Wherein 1 is the substrate, 2 is the sample zone, 3 is the labeling zone, 4 is the detection zone, 5 to 7 are sub-detection zones, 8 is the quality control zone, and 9 is the absorbent zone.

FIG. 3 shows a sensitivity comparison between the fluorescence immunochromatographic method and the colloidal gold method at a coating concentration of 2 mg/mL.

## SEQUENCE DESCRIPTION

SEQ ID NO: 1 is the amino acid sequence of the MCV, where X represents citrulline (Citrulline, Cit).

SEQ ID NO: 2 is the amino acid sequence of the CCP, where a disulfide bond exists between C3 and C16, and X represents citrulline (Citrulline, Cit).

SEQ ID NO: 1

MSTXSVSSSSYRGMFGXPGTASGPSSSXSYVTTSTRTYSLXSALGPSTSXSLYASSPGRVYAT
GSSYVXLRSSVPXVGLLQDSVDFSLADAINTEFKNTRTNEKVELQELNDRFANYIDKVRFLE
QQNKILLAELEQLKGQGKSXLGDLYEEEMRELRXQVDQLTNDKARVEVERDNLAEDIMXL
REKLQEEMLQREEAENTLQSFRQDVDNASLAXLDLERKVESLQEEIAFLKKLHEEEIQELQ
AQIQEQHVQIDVDVSKPDLTAALRDVRQQYESVAAKNLQEAEEWYKSKFADLSEAANRNN
DALRQAKQESTEYRRQVQSLTCEVDALKGTNESLERQMREMEENFAVEAANYQDTIGGLQ
DEIQNMKEEMAGHLREYQDLLNVKMALDIEIATYGKLLEGEESRISLPLPNFSSLNLGETNL
DSLPLVDTHSKXTLLIKTVETRDRQVINETSQHXDDLEGHHHHHH

SEQ ID NO: 2
HQCHQSSTGXHRHGRCGRTGS

## DETAILED DESCRIPTION OF THE INVENTION

One object of the present application is to simultaneously detect anti-mutant citrullinated vimentin antibodies (Anti-MCV), anti-cyclic citrullinated peptide antibodies (Anti-CCP), and rheumatoid factor (RF) using the principle of fluorescence immunochromatography.

Anti-MCV detection uses the principle of the indirect detection method. The Anti-MCV in the sample to be detected first binds to the fluorescent material coupled to goat anti-human IgG on the sample loading zone, and captured by recombinant MCV (T-line, which may be T1 line, T2 line, or T3 line) coated on the detection zone (e.g., a nitrocellulose membrane) by chromatographic action. The T-line fluorescent signal can be quantitatively detected by a matching instrument. The fluorescence intensity is positively correlated with the concentration of Anti-MCV in the sample.

Anti-CCP detection uses the principle of the indirect detection method. The Anti-CCP in the sample to be detected first binds to the fluorescent material coupled to goat anti-human IgG on the sample loading zone, and captured by recombinant CCP (T-line, which may be T1 line, T2 line, or T3 line) coated on the detection zone (e.g., a nitrocellulose membrane) by chromatographic action. The T-line fluorescent signal can be quantitatively detected by a matching instrument. The fluorescence intensity is positively correlated with the concentration of Anti-CCP in the sample, and the concentration of Anti-CCP in the sample can be quantitatively displayed by instrument calculation.

RF detection uses the principle of the double-antigen sandwich method. The RF in the sample to be detected first binds to the fluorescent material coupled to denatured human IgG on the sample loading zone, and captured by a denatured human IgG (T-line, which may be T1 line, T2 line, or T3 line) coated on the detection zone (e.g., a nitrocellulose membrane) by chromatographic action, and the T-line fluorescent signal can be quantitatively detected by a matching instrument, the fluorescence intensity is positively correlated with the RF concentration in the sample, and the concentration of RF in the sample can be quantitatively displayed by instrument calculation.

A first aspect of the present application provides a fluorescent immunochromatographic test strip for detecting rheumatoid arthritis, the test strip includes a sample loading zone, a detection zone, and an absorbent zone; the detection zone includes a first sub-detection zone coated with MCV, a second sub-detection zone coated with CCP, and a third sub-detection zone coated with denatured human IgG; wherein the coating amount of the MCV, the CCP, or the denatured human IgG is independently 0.15-0.75 μg; the amino acid sequence of the MCV is set forth in SEQ ID NO: 1; the amino acid sequence of the CCP is set forth in SEQ ID NO: 2; the sample loading zone contains fluorescent material-labeled goat anti-human IgG, fluorescent material-labeled denatured human IgG, and fluorescent material-labeled goat anti-rabbit IgG, in a mass ratio of 20:10:1.

In some embodiments, the fluorescent material-labeled goat anti-human IgG, the fluorescent material-labeled denatured human IgG, and the fluorescent material-labeled goat anti-rabbit IgG are mixed and then sprayed to the pad at 4 μL/cm.

In some embodiments, the sample loading zone, the detection zone, and the absorbent zone are arranged sequentially from upstream to downstream, according to the flow direction of the sample on the test strip.

In some embodiments, the coating concentration of the MCV, the CCP, and the denatured human IgG is 0.5-1.5 mg/mL, with a streak at 1.0 μL/cm.

In some embodiments, the first sub-detection zone, the second sub-detection zone, and the third sub-detection zone are sequentially arranged sequentially from upstream to downstream, according to the flow direction of the sample on the test strip.

In some embodiments, the first sub-detection zone, the third sub-detection zone, and the second sub-detection zone are sequentially arranged sequentially from upstream to downstream, according to the flow direction of the sample on the test strip.

In some embodiments, the second sub-detection zone, the first sub-detection zone, and the third sub-detection zone are sequentially arranged sequentially from upstream to downstream, according to the flow direction of the sample on the test strip.

In some embodiments, the second sub-detection zone, the third sub-detection zone, and the first sub-detection zone are sequentially arranged sequentially from upstream to downstream, according to the flow direction of the sample on the test strip.

In some embodiments, the third sub-detection zone, the first sub-detection zone, and the second sub-detection zone are sequentially arranged sequentially from upstream to downstream, according to the flow direction of the sample on the test strip.

In some embodiments, the third sub-detection zone, the second sub-detection zone, and the first sub-detection zone are sequentially arranged sequentially from upstream to downstream, according to the flow direction of the sample on the test strip.

In some embodiments, the coating amount of the MCV is 0.15 μg, 0.155 μg, 0.16 μg, 0.165 μg, 0.17 μg, 0.175 μg, 0.18 μg, 0.185 μg, 0.19 μg, 0.195 μg, 0.2 μg, 0.205 μg, 0.21 μg, 0.215 μg, 0.22 μg, 0.225 μg, 0.23 μg, 0.235 μg, 0.24 μg, 0.245 μg, 0.25 μg, 0.255 μg, 0.26 μg, 0.265 μg, 0.27 μg, 0.275 μg, 0.28 μg, 0.285 μg, 0.29 μg, 0.295 μg, 0.3 μg, 0.305 μg, 0.31 μg, 0.315 μg, 0.32 μg, 0.325 μg, 0.33 μg, 0.335 μg, 0.34 μg, 0.345 μg, 0.35 μg, 0.355 μg, 0.36 μg, 0.365 μg, 0.37 μg, 0.375 μg, 0.38 μg, 0.385 μg, 0.39 μg, 0.395 μg, 0.4 μg, 0.405 μg, 0.41 μg, 0.415 μg, 0.42 μg, 0.425 μg, 0.43 μg, 0.435 μg, 0.44 μg, 0.445 μg, 0.45 μg, 0.455 μg, 0.46 μg, 0.465 μg, 0.47 μg, 0.475 μg, 0.48 μg, 0.485 μg, 0.49 μg, 0.495 μg, 0.5 μg, 0.505 μg, 0.51 μg, 0.515 μg, 0.52 μg, 0.525 μg, 0.53 μg, 0.535 μg, 0.54 μg, 0.545 μg, 0.55 μg, 0.555 μg, 0.56 μg, 0.565 μg, 0.57 μg, 0.575 μg, 0.58 μg, 0.585 μg, 0.59 μg, 0.595 μg, 0.6 μg, 0.605 μg, 0.61 μg, 0.615 μg, 0.62 μg, 0.625 μg, 0.63

μg, 0.635 μg, 0.64 μg, 0.645 μg, 0.65 μg, 0.655 μg, 0.66 μg, 0.665 μg, 0.67 μg, 0.675 μg, 0.68 μg, 0.685 μg, 0.69 μg, 0.695 μg, 0.7 μg, 0.705 μg, 0.71 μg, 0.715 μg, 0.72 μg, 0.725 μg, 0.73 μg, 0.735 μg, 0.74 μg, 0.745 μg, 0.75 μg, or any range between the above values.

In some embodiments, the coating concentration of the MCV is 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, or any range between the above values.

In some embodiments, the coating amount of the CCP is 0.15 μg, 0.155 μg, 0.16 μg, 0.165 μg, 0.17 μg, 0.175 μg, 0.18 μg, 0.185 μg, 0.19 μg, 0.195 μg, 0.2 μg, 0.205 μg, 0.21 μg, 0.215 μg, 0.22 μg, 0.225 μg, 0.23 μg, 0.235 μg, 0.24 μg, 0.245 μg, 0.25 μg, 0.255 μg, 0.26 μg, 0.265 μg, 0.27 μg, 0.275 μg, 0.28 μg, 0.285 μg, 0.29 μg, 0.295 μg, 0.3 μg, 0.305 μg, 0.31 μg, 0.315 μg, 0.32 μg, 0.325 μg, 0.33 μg, 0.335 μg, 0.34 μg, 0.345 μg, 0.35 μg, 0.355 μg, 0.36 μg, 0.365 μg, 0.37 μg, 0.375 μg, 0.38 μg, 0.385 μg, 0.39 μg, 0.395 μg, 0.4 μg, 0.405 μg, 0.41 μg, 0.415 μg, 0.42 μg, 0.425 μg, 0.43 μg, 0.435 μg, 0.44 μg, 0.445 μg, 0.45 μg, 0.455 μg, 0.46 μg, 0.465 μg, 0.47 μg, 0.475 μg, 0.48 μg, 0.485 μg, 0.49 μg, 0.495 μg, 0.5 μg, 0.505 μg, 0.51 μg, 0.515 μg, 0.52 μg, 0.525 μg, 0.53 μg, 0.535 μg, 0.54 μg, 0.545 μg, 0.55 μg, 0.555 μg, 0.56 μg, 0.565 μg, 0.57 μg, 0.575 μg, 0.58 μg, 0.585 μg, 0.59 μg, 0.595 μg, 0.6 μg, 0.605 μg, 0.61 μg, 0.615 μg, 0.62 μg, 0.625 μg, 0.63 μg, 0.635 μg, 0.64 μg, 0.645 μg, 0.65 μg, 0.655 μg, 0.66 μg, 0.665 μg, 0.67 μg, 0.675 μg, 0.68 μg, 0.685 μg, 0.69 μg, 0.695 μg, 0.7 μg, 0.705 μg, 0.71 μg, 0.715 μg, 0.72 μg, 0.725 μg, 0.73 μg, 0.735 μg, 0.74 μg, 0.745 μg, 0.75 μg, or any range between the above values. In some embodiments, the coating concentration of the CCP is 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, or any range between the above values.

In some embodiments, the coating amount of the denatured human IgG is 0.15 μg, 0.155 μg, 0.16 μg, 0.165 μg, 0.17 μg, 0.175 μg, 0.18 μg, 0.185 μg, 0.19 μg, 0.195 μg, 0.2 μg, 0.205 μg, 0.21 μg, 0.215 μg, 0.22 μg, 0.225 μg, 0.23 μg, 0.235 μg, 0.24 μg, 0.245 μg, 0.25 μg, 0.255 μg, 0.26 μg, 0.265 μg, 0.27 μg, 0.275 μg, 0.28 μg, 0.285 μg, 0.29 μg, 0.295 μg, 0.3 μg, 0.305 μg, 0.31 μg, 0.315 μg, 0.32 μg, 0.325 μg, 0.33 μg, 0.335 μg, 0.34 μg, 0.345 μg, 0.35 μg, 0.355 μg, 0.36 μg, 0.365 μg, 0.37 μg, 0.375 μg, 0.38 μg, 0.385 μg, 0.39 μg, 0.395 μg, 0.4 μg, 0.405 μg, 0.41 μg, 0.415 μg, 0.42 μg, 0.425 μg, 0.43 μg, 0.435 μg, 0.44 μg, 0.445 μg, 0.45 μg, 0.455 μg, 0.46 μg, 0.465 μg, 0.47 μg, 0.475 μg, 0.48 μg, 0.485 μg, 0.49 μg, 0.495 μg, 0.5 μg, 0.505 μg, 0.51 μg, 0.515 μg, 0.52 μg, 0.525 μg, 0.53 μg, 0.535 μg, 0.54 μg, 0.545 μg, 0.55 μg, 0.555 μg, 0.56 μg, 0.565 μg, 0.57 μg, 0.575 μg, 0.58 μg, 0.585 μg, 0.59 μg, 0.595 μg, 0.6 μg, 0.605 μg, 0.61 μg, 0.615 μg, 0.62 μg, 0.625 μg, 0.63 μg, 0.635 μg, 0.64 μg, 0.645 μg, 0.65 μg, 0.655 μg, 0.66 μg, 0.665 μg, 0.67 μg, 0.675 μg, 0.68 μg, 0.685 μg, 0.69 μg, 0.695 μg, 0.7 μg, 0.705 μg, 0.71 μg, 0.715 μg, 0.72 μg, 0.725 μg, 0.73 μg, 0.735 μg, 0.74 μg, 0.745 μg, 0.75 μg, or any range between the above values.

In some embodiments, the coating concentration of denatured human IgG is 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, or any range between the above values.

In some embodiments, the denaturation condition of denatured human IgG is a water bath at 60°C for 30 minutes.

In some embodiments, fluorescent materials include, but are not limited to, fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a substrate.

In some embodiments, the fluorescent immunochromatographic test strip further comprises a quality control zone located downstream of the first sub-detection zone, the second sub-detection zone, or the third sub-detection zone in the flow direction of the samples on the test strip.

In some embodiments, the quality control zone is coated with rabbit IgG.

In some embodiments, the coating concentration of the rabbit IgG is 0.5-1.5 mg/mL.

In some embodiments, the coating amount of the rabbit IgG is 0.15 μg, 0.155 μg, 0.16 μg, 0.165 μg, 0.17 μg, 0.175 μg, 0.18 μg, 0.185 μg, 0.19 μg, 0.195 μg, 0.2 μg, 0.205 μg, 0.21 μg, 0.215 μg, 0.22 μg, 0.225 μg, 0.23 μg, 0.235 μg, 0.24 μg, 0.245 μg, 0.25 μg, 0.255 μg, 0.26 μg, 0.265 μg, 0.27 μg, 0.275 μg, 0.28 μg, 0.285 μg, 0.29 μg, 0.295 μg, 0.3 μg, 0.305 μg, 0.31 μg, 0.315 μg, 0.32 μg, 0.325 μg, 0.33 μg, 0.335 μg, 0.34 μg, 0.345 μg, 0.35 μg, 0.355 μg, 0.36 μg, 0.365 μg, 0.37 μg, 0.375 μg, 0.38 μg, 0.385 μg, 0.39 μg, 0.395 μg, 0.4 μg, 0.405 μg, 0.41 μg, 0.415 μg, 0.42 μg, 0.425 μg, 0.43 μg, 0.435 μg, 0.44 μg, 0.445 μg, 0.45 μg, 0.455 μg, 0.46 μg, 0.465 μg, 0.47 μg, 0.475 μg, 0.48 μg, 0.485 μg, 0.49 μg, 0.495 μg, 0.5 μg, 0.505 μg, 0.51 μg, 0.515 μg, 0.52 μg, 0.525 μg, 0.53 μg, 0.535 μg, 0.54 μg, 0.545 μg, 0.55 μg, 0.555 μg, 0.56 μg, 0.565 μg, 0.57 μg, 0.575 μg, 0.58 μg, 0.585 μg, 0.59 μg, 0.595 μg, 0.6 μg, 0.605 μg, 0.61 μg, 0.615 μg, 0.62 μg, 0.625 μg, 0.63 μg, 0.635 μg, 0.64 μg, 0.645 μg, 0.65 μg, 0.655 μg, 0.66 μg, 0.665 μg, 0.67 μg, 0.675 μg, 0.68 μg, 0.685 μg, 0.69 μg, 0.695 μg, 0.7 μg, 0.705 μg, 0.71 μg, 0.715 μg, 0.72 μg, 0.725 μg, 0.73 μg, 0.735 μg, 0.74 μg, 0.745 μg, 0.75 μg, or any range between the above values.

In some embodiments, the coating concentration of the rabbit IgG is 0.5 mg/mL, 0.6 mg/mL, 0.7 mg/mL, 0.8 mg/mL, 0.9 mg/mL, 1.0 mg/mL, 1.1 mg/mL, 1.2 mg/mL, 1.3 mg/mL, 1.4 mg/mL, 1.5 mg/mL, or any range between the above values.

The fluorescent material of the goat anti-rabbit IgG coated onto the sample loading zone can be captured by chromatographic action on the rabbit IgG (C-line, control line) coated on the detection zone (e.g., a nitrocellulose membrane). The C-line fluorescent signal can be quantitatively detected by a matching instrument, thereby achieving quality control.

A second aspect of the present application provides a kit comprising the fluorescent immunochromatographic test strip of the first aspect and instructions.

In some embodiments, the fluorescent immunochromatographic test strip further comprises a substrate.

In some embodiments, the sample loading zone is divided into a sample zone for receiving the sample, and a labeling zone having fluorescent material-labeled goat anti-human IgG, fluorescent material-labeled denatured human IgG, and fluorescent material-labeled goat anti-rabbit IgG coated thereon.

In some embodiments, the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a quality control zone, which is located downstream of the first detection zone, the second detection zone, or the third detection zone in the flow direction of the sample on the test strip.

In some embodiments, the quality control zone is coated with rabbit IgG, with a coating amount of 0.15-0.75 μg.

In some embodiments, the sample loading zone, the detection zone, and the absorbent zone are arranged sequentially from upstream to downstream, according to the flow direction of the sample on the test strip.

In some embodiments, the coating concentration of MCV, CCP, and denatured human IgG is 0.5-1.5 mg/mL.

A third aspect of the present application provides the use of the fluorescent immunochromatographic test strip of the first aspect in the preparation of a kit for detecting rheumatoid arthritis.

In some embodiments, the fluorescent immunochromatographic test strip further comprises a substrate.

In some embodiments, the sample loading zone is divided into a sample zone for receiving the sample, and a labeling zone having fluorescent material-labeled goat anti-human IgG, fluorescent material-labeled denatured human IgG, and fluorescent material-labeled goat anti-rabbit IgG coated thereon.

In some embodiments, the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a quality control zone, which is located downstream of the first detection zone, the second detection zone, or the third detection zone in the flow direction of the sample on the test strip.

In some embodiments, the quality control zone is coated with rabbit IgG, with a coating amount of 0.15-0.75 μg.

In some embodiments, the sample loading zone, the detection zone, and the absorbent zone are arranged sequentially from upstream to downstream, according to the flow direction of the sample on the test strip.

In some embodiments, the coating concentration of MCV, CCP, and denatured human IgG is 0.5-1.5 mg/mL.

A fourth aspect of the present application provides a method of diagnosing, auxiliary diagnosing, detecting, or assessing whether a subject suffers from rheumatoid arthritis, the severity of rheumatoid arthritis, the stage of rheumatoid arthritis, or the risk of developing rheumatoid arthritis, comprising the steps of:

a. adding a blood sample from the subject to the sample loading zone of the fluorescent immunochromatographic test strip;

the fluorescent immunochromatographic test strip includes a sample loading zone, a detection zone, and an absorbent zone;
detection zone includes a first sub-detection zone coated with MCV, a second sub-detection zone coated with CCP, and a third sub-detection zone coated with denatured human IgG;
wherein the coating amount of the MCV, the CCP, or the denatured human IgG is independently 0.15-0.75 μg;
the amino acid sequence of the MCV is set forth in SEQ ID NO: 1;
the amino acid sequence of the CCP is set forth in SEQ ID NO: 2;
the sample loading zone contains a fluorescent material-labeled goat anti-human IgG, fluorescent material-labeled denatured human IgG, and fluorescent material-labeled goat anti-rabbit IgG, in a mass ratio of 20:10:1; and

b. determining qualitative or quantitative results generated from the fluorescent materials in the first sub-detection zone, the second sub-detection zone, and the third sub-detection zone, and assessing whether the subject suffers

**EP 4 745 574 A1**

from rheumatoid arthritis, the severity of rheumatoid arthritis (disease severity), the stage of rheumatoid arthritis, or the risk of developing rheumatoid arthritis based on the qualitative or quantitative results.

In some embodiments, in step (b), when the first sub-detection zone, the second sub-detection zone, and the third sub-detection zone show positive fluorescence results, the subject is diagnosed with rheumatoid arthritis.

In some embodiments, based on the qualitative or quantitative fluorescence results from the first sub-detection zone and the second sub-detection zone, evaluates whether a subject suffers from rheumatoid arthritis, the severity of rheumatoid arthritis, the stage of rheumatoid arthritis, or the risk of developing rheumatoid arthritis.

In some embodiments, based on the qualitative or quantitative fluorescence results from the first sub-detection zone and the third sub-detection zone, evaluates whether a subject suffers from rheumatoid arthritis, the severity of rheumatoid arthritis, the stage of rheumatoid arthritis, or the risk of developing rheumatoid arthritis.

In some embodiments, based on the qualitative or quantitative fluorescence results from the second sub-detection zone and the third sub-detection zone, evaluates whether a subject suffers from rheumatoid arthritis, the severity of rheumatoid arthritis, the stage of rheumatoid arthritis, or the risk of developing rheumatoid arthritis.

In some embodiments, based on the qualitative or quantitative fluorescence results from the first sub-detection zone, evaluates whether a subject suffers from rheumatoid arthritis, the severity of rheumatoid arthritis, the stage of rheumatoid arthritis, or the risk of developing rheumatoid arthritis.

In some embodiments, based on the qualitative or quantitative fluorescence results from the second sub-detection zone, evaluates whether a subject suffers from rheumatoid arthritis, the severity of rheumatoid arthritis, the stage of rheumatoid arthritis, or the risk of developing rheumatoid arthritis.

In some embodiments, based on the qualitative or quantitative fluorescence results from the third sub-detection zone, evaluates whether a subject suffers from rheumatoid arthritis, the severity of rheumatoid arthritis, the stage of rheumatoid arthritis, or the risk of developing rheumatoid arthritis.

In some embodiments, if the first sub-detection zone, the second sub-detection zone, and the third sub-detection zone show fluorescently positive results, it is estimated that the donor providing the blood sample has a 99.2% probability of suffering from rheumatoid arthritis.

In some embodiments, if the first sub-detection zone and the second sub-detection zone show fluorescently positive results, it is estimated that the donor providing the blood sample has a 94.6% probability of suffering from rheumatoid arthritis.

In some embodiments, if the first sub-detection zone and the third sub-detection zone show fluorescently positive results, it is estimated that the donor providing the blood sample has a 91.7% probability of suffering from rheumatoid arthritis.

In some embodiments, if the second sub-detection zone and the third sub-detection zone show fluorescently positive results, it is estimated that the donor providing the blood sample has a 93.6% probability of suffering from rheumatoid arthritis.

In some embodiments, if the first sub-detection zone shows a fluorescence positive result (or a value greater than 20 U/mL), it is estimated that the donor providing the blood sample has an 88.5% probability of suffering from rheumatoid arthritis.

In some embodiments, if the second sub-detection zone shows a fluorescence positive result (or a value greater than 25 U/mL), it is estimated that the donor providing the blood sample has a 92.3% probability of suffering from rheumatoid arthritis.

In some embodiments, if the third sub-detection zone shows a fluorescence positive result (or a value greater than 20 IU/mL), it is estimated that the donor providing the blood sample has a 78.8% probability of suffering from rheumatoid arthritis.

According to the X-ray staging criteria, RA patients are divided into a non-bone erosion group (stage I) and a bone erosion group (stages II-IV).

Stage I: joint non-destructive changes or osteoporosis at the joint ends.

Stage II: osteoporosis at the joint ends, with mild subarticular cystic destruction or bone erosion changes; limited joint movement without deformities; muscular atrophy around adjacent joints; extraarticular soft tissue lesions.

Stage III: significant osteoporosis with articular cartilage or bone destruction; joint space narrowing, joint subluxation, etc.; widespread muscular atrophy; extraarticular soft tissue lesions.

Stage IV: fibrous or bony ankylosis in addition to the changes seen in stages II and III.

In some embodiments, if the first test line measurement is in the range of 108.1-334.62 U/mL and the second test line measurement is in the range of 54.22-239.08 U/mL, it is estimated that the condition of the donor providing the blood sample is in stage I according to the X-ray staging criteria.

In some embodiments, if the first test line measurement is in the range of 209.69-466.65 U/mL and the second test line measurement is in the range of 192.78-449.86 U/mL, it is estimated that the condition of the donor providing the blood sample is at stage II according to the X-ray staging criteria.

8

In some embodiments, if the first test line measurement is in the range of 215.81-480.71U/mL and the second test line measurement is in the range of 262.96-601.94 U/mL, it is estimated that the condition of the donor providing the blood sample is stage III according to the X-ray staging criteria.

In some embodiments, if the first test line measurement is in the range of 129.97-333.55 U/mL and the second test line measurement is in the range of 572.11-1111.47 U/mL, it is estimated that the condition of the donor providing the blood sample is in stage IV according to the X-ray staging criteria.

In some embodiments, the first test line measurement, the second test line measurement, or the third test line measurement is calibrated using a calibration curve.

In some embodiments, the first test line measurement, the second test line measurement, or the third test line measurement is a quantitative result calculated from the fluorescence value using a calibration curve.

In some embodiments, the blood sample is a whole blood sample, a plasma sample, or a serum sample.

In some embodiments, the fluorescent immunochromatographic test strip further comprises a substrate.

In some embodiments, the sample loading zone is divided into a sample zone for receiving the sample, and a labeling zone having fluorescent material-labeled goat anti-human IgG, fluorescent material-labeled denatured human IgG, and fluorescent material-labeled goat anti-rabbit IgG coated thereon.

In some embodiments, the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a quality control zone, which is located downstream of the first detection zone, the second detection zone, or the third detection zone in the flow direction of the sample on the test strip.

In some embodiments, the quality control zone is coated with rabbit IgG, with a coating amount of 0.15-0.75 μg.

In some embodiments, the sample loading zone, the detection zone, and the absorbent zone are arranged sequentially from upstream to downstream, according to the flow direction of the sample on the test strip.

In some embodiments, the coating concentration of the MCV, the CCP, and the denatured human IgG is 0.5-1.5 mg/mL.

In some embodiments, the fluorescent immunochromatographic test strip is used for diagnosing, auxiliary diagnosing, detecting, or assessing whether a subject suffers from rheumatoid arthritis, the severity of rheumatoid arthritis, the stage of rheumatoid arthritis, or the risk of developing rheumatoid arthritis.

In some embodiments, the fluorescent qualitative results of the fluorescent immunochromatographic test strip are used for assessing whether a subject suffers from rheumatoid arthritis.

In some embodiments, the fluorescent quantitative results of the fluorescent immunochromatographic test strips are used for assessing the severity of rheumatoid arthritis in a subject.

A fifth aspect of the present application provides a method for detecting the content of the anti-mutant citrullinated vimentin antibody (Anti-MCV), the anti-cyclic citrullinated peptide antibody (Anti-CCP), and the rheumatoid factor (RF) in a biological sample from a subject, comprising the steps of:

a. adding a biological sample from the subject to the sample loading zone of the fluorescent immunochromatographic test strip;

the fluorescent immunochromatographic test strip includes a sample loading zone, a detection zone, and an absorbent zone;
detection zone includes a first sub-detection zone coated with MCV, a second sub-detection zone coated with CCP, and a third sub-detection zone coated with denatured human IgG;
wherein the coating amount of the MCV, the CCP, or the denatured human IgG is independently 0.15-0.75 μg;
the amino acid sequence of the MCV is set forth in SEQ ID NO: 1;
the amino acid sequence of the CCP is set forth in SEQ ID NO: 2;
the sample loading zone contains a fluorescent material-labeled goat anti-human IgG, fluorescent material-labeled denatured human IgG, and fluorescent material-labeled goat anti-rabbit IgG, in a mass ratio of 20:10:1; and

b. determining qualitative or quantitative results generated from the fluorescent materials in the first sub-detection zone, the second sub-detection zone, and the third sub-detection zone, and determining the content of the anti-mutant citrullinated vimentin antibody (Anti-MCV), the anti-cyclic citrullinated peptide antibody (Anti-CCP), and the rheumatoid factor (RF) in the biological sample based on the quantitative result.

In some embodiments, the biological sample is a whole blood sample, a plasma sample, or a serum sample.

In some embodiments, the fluorescent immunochromatographic test strip further comprises a substrate.

In some embodiments, the sample loading zone is divided into a sample zone for receiving the sample, and a labeling zone having fluorescent material-labeled goat anti-human IgG, fluorescent material-labeled denatured human IgG, and fluorescent material-labeled goat anti-rabbit IgG coated thereon.

In some embodiments, the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

In some embodiments, the fluorescent immunochromatographic test strip further comprises a quality control zone located downstream of the first detection zone, the second detection zone, and the third detection zone in the flow direction of the sample on the test strip.

In some embodiments, the quality control zone is coated with rabbit IgG, with a coating amount of 0.15-0.75 $\mu$g.

In some embodiments, the sample loading zone, the detection zone, and the absorbent zone are arranged sequentially from upstream to downstream, according to the flow direction of the sample on the test strip.

In some embodiments, the coating concentration of the MCV, the CCP, and the denatured human IgG is 0.5-1.5 mg/mL.

## Example

The present application will be described in more detail with specific examples. The following examples are provided for illustrative purposes only and are not intended to limit the present application in any way. Those skilled in the art will readily recognize various non-critical parameters that may be altered or modified to produce substantially the same result.

## Example 1: Preparation of fluorescent immunochromatographic test strips for the quantitative combined detection of Anti-MCV antibody, Anti-CCP antibody, and RF

(a) Treatment of fluorescent material:
Fluorescent material was dissolved in a phosphoric acid buffer (PB) solution having a pH of 8.0. After the fluorescent material was sufficiently resuspended, an activator, 1-(3-dimethylaminopropyl)-3-ethylcarbodiimide (EDC), was added, mixed thoroughly, followed by the addition of a stabilizer, N-hydroxysuccinimide (NHS), in an amount equal to the EDC. The mixture was then incubated at room temperature for 2 hours. Then, the mixture was centrifuged at 14,000 rpm for 30 min. The supernatant was discarded, and the precipitate was washed with a phosphoric acid buffer (PB) solution at pH 7.0 and redissolved. The concentrations of both EDC and NHS were 10 mg/mL;

(b) Antibody labeling:

Goat anti-human IgG was added to the fluorescent material obtained in step (a), mixed thoroughly, and stirred continuously. It was incubated at 4°C overnight for the reaction. After the reaction was complete, the mixture was centrifuged at 14,000 rpm for 30 min. The supernatant was discarded, and the precipitate was washed with a phosphoric acid buffer (PB) solution at pH 7.0 and redissolved to obtain the fluorescent material-labeled goat anti-human IgG;

Denatured human IgG was added to the fluorescent material solution obtained in step (a), mixed thoroughly, and stirred continuously. It was incubated at 4°C overnight for the reaction. After the reaction was complete, the mixture was centrifuged at 14,000 rpm for 30 min. The supernatant was discarded, and the precipitate was washed with a phosphoric acid buffer (PB) solution at pH 7.0 and redissolved to obtain the fluorescent material-labeled denatured human IgG;

Goat anti-rabbit IgG was added to the fluorescent material solution obtained in step (a) , mixed thoroughly, and stirred continuously. It was incubated at 4°C overnight for the reaction. After the reaction was complete, the mixture was centrifuged at 14,000 rpm for 30 min. The supernatant was discarded, and the precipitate was washed with a phosphoric acid buffer (PB) solution at pH 7.0 and redissolved to obtain the fluorescent material-labeled goat anti-rabbit IgG;

(c) Preparation of the sample loading zone: the fluorescent material-labeled goat anti-human IgG, the fluorescent material-labeled denatured human IgG, and the fluorescent material-labeled goat anti-rabbit IgG obtained in step (b) were coated onto the sample loading zone in a ratio of 20:10:1, and then dried at 37°C for 24 h. The coating concentration of the fluorescent material-labelled goat anti-human IgG was 2% (volume percent v/v, i.e., the ratio of the volume of the fluorescent material-labelled goat anti-human IgG to the total volume of the preparation), and the mixture was sprayed onto the pad at 4 μL/cm after mixing with the other two labeled materials. The coating concentration of fluorescent material-labeled denatured human IgG was 1%, and the mixture was sprayed onto the pad at 4 μL/cm after mixing with the other two labeled materials. The coating concentration of the fluorescent material-labeled goat anti-rabbit IgG was 0.1%, and the mixture was sprayed onto the pad at 4 μL/cm after mixing with the other two labeled materials.

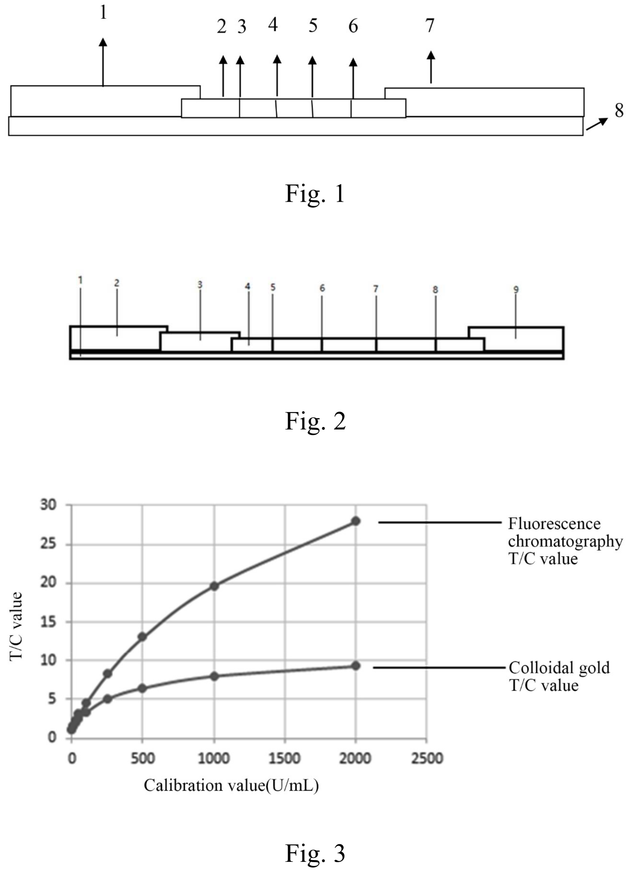

(d) Preparation of the detection zone (2) (as exemplified in FIG. 1):

Preparation of the test lines: recombinant MCV (as set forth in SEQ ID NO: 1), recombinant CCP (as set forth in SEQ ID NO: 2), and denatured human IgG were each diluted to a concentration of 1 mg/ml. The first sub-test line (3), the second sub-test line (4), and the third sub-test line (5) were streaked onto the detection zone at 1.0 μL/cm, respectively;

The streaking amount was 1.0 μL/cm, the solution concentration was 1 mg/ml, the strip width was 0.4 cm, and the amount of protein coated on the test line was approximately 0.4 μg;

Preparation of the control line (6): the rabbit IgG, at a concentration of 1 mg/mL, was streaked onto the control line (6) on the detection zone at 1 μL/cm;

The coated detection zone (nitrocellulose membrane) mentioned above was dried at 37°C for 24 h. After drying, the nitrocellulose membrane was removed and subjected to sealed drying and storage;

(e) Assembly:

The chromatographic film (2) obtained in step (d) was attached to the substrate (8), and then the sample loading zone (1) was attached to the end of the chromatographic film (2) near the first test line (3). The absorbent paper (7) was attached to the end of the chromatographic film (2) near the control line (6). The adhered substrate (8) was cut into test strips with a length of 3.5 mm, placed into the plastic case, pressed, and assembled into the kit in an environment where the humidity is less than 30%.

**Example 2: Another preparation method for fluorescent immunochromatographic test strips for the quantitative combined detection of Anti-MCV antibody, Anti-CCP antibody, and RF (as exemplified in FIG. 2)**

1) PVC plate (1): the surface of the plate was coated with a non-dry adhesive, which functions to provide support and fixation.

2) Sample zone (2): the sample zone was composed of glass fibers and subjected to a spraying treatment with a solution containing buffer, anti-red blood cell antibody, surfactant, sugar, and other components, which functions to separate interfering substances, such as red blood cells, from the buffer.

3) Labeling zone (3): the labeling zone was composed of glass fiber polyester or the like and was subjected to a spraying treatment with a solution containing fluorescent microsphere marker of goat antibody IgG, fluorescent microsphere marker of denatured human IgG, and fluorescent microsphere marker of goat anti-rabbit IgG.

4) Detection zone (4): the detection zone was a nitrocellulose membrane, which functions as a carrier for a chromatographic reaction, with a first test line, a second test line, a third test line, and a control line sequentially coated thereon.

5) First test line (5, 6, or 7): the first test line was coated on the NC membrane and contained recombinant MCV protein (as set forth in SEQ ID NO: 1), which functions to detect anti-MCV antibodies.

6) Second test line (5, 6, or 7), the second test line was coated on the NC membrane and contained recombinant CCP protein (as set forth in SEQ ID NO: 2), which functions to detect anti-CCP antibodies.

7) Third test line (5, 6, or 7): the third test line was coated on NC membrane and contained denatured human IgG), which functions to detect RF.

8) Control line (8): the control line was coated on the NC membrane and contained rabbit IgG, which functions to indicate the effectiveness of the product.

9) Absorbent pad (9): the absorbent pad is composed of plant fibers, cotton lint, or the like, which functions to absorb excess liquid and provide power for the chromatographic reaction.

**Example 3: Preparation of fluorescent immunochromatographic test strips of Anti-MCV antibody, Anti-CCP antibody, and RF (quantum dot immunochromatographic test strips).**

By embedding the quantum dot microspheres in the inorganic material, the quantum dots were not easily affected by the external environment, and the stability and detection sensitivity of the quantum dots were significantly improved.

The surface of the quantum dot microspheres was hydrophilically coated, with good biocompatibility and low non-specific adsorption, making them excellent material for high-sensitivity biological detection. As a type of fluorescent microsphere, the quantum dot microspheres were assembled from the nanometer to the micron scale, thereby protecting the quantum dots. Their surface can be easily modified with functional groups such as carboxyl (-COOH), amino (-NH2), p-toluenesulfonyl (-Ts), and streptavidin (-SA).

The method for preparing the quantum dot immunochromatographic test strips is as follows:

### (a) Reagents

1) Carboxyl-modified quantum dot microspheres
2) Goat anti-human IgG, denatured human IgG, goat anti-rabbit IgG
3) Activators, EDC, Sulfo-NHS
4) Activating solution Buffer A (0.05 M/L of MES, pH 6.0)
5) Reaction solution Buffer B (0.05 M/L of MES, pH 6.5)
6) Blocking solution Buffer C (0.05 M/L of TRIS-HCl + 1% W/V BSA, pH 8.0)
7) Storage solution Buffer D (0.05 M/L of TRIS-HCl + 1% W/V BSA + 10% W/V trehalose, pH 8.0)

### (b) Marking process

1% quantum dot microspheres can be diluted to different concentrations, such as 10-fold, 5-fold, or original concentrations, for labeling, that is, the microsphere concentration during the reaction can be 1 mg/mL, 2 mg/mL, or 10 mg/mL. The sensitivity differences at the three concentrations were not significant, but the original concentration label tends to aggregate easily. If the concentration exceeds 2 mg/mL, the microsphere concentration is high, making it difficult to observe the dispersion during the reaction process. Therefore, a 10-fold dilution is preferred, with a microsphere concentration of 1 mg/mL.

1) Washing microspheres
50 $\mu$L of 1% quantum dot microspheres was added to a 1.5 mL centrifuge tube, followed by the addition of 450 $\mu$L of activating solution Buffer A. The mixture was shaken at 1000 rpm for 1 min, then centrifuged at 14,800 rpm for 30 min. The supernatant was removed, 400 $\mu$L of activating solution Buffer A was added, and the mixture was redissolved and washed once.
2) Activated microspheres
10 mg/mL EDC and Sulfo-NHS solutions were prepared using the activating solution Buffer A. For example, 1 mg of EDC was weighed and dissolved in 100 $\mu$L of Buffer A. 50 $\mu$L of Sulfo-NHS solution and 50 $\mu$L of EDC solution were successively added to the washed microspheres, that is, EDC and NHS used at concentrations of 1 mg/mL. The mixture was then placed on a vortex shaker and shaken at 1000 rpm for 30 min for activation.
3) Post-activation washing
After activation, the mixture was washed, and the solution was replaced with the reaction solution Buffer B:

(1) After activation, the mixture was centrifuged at 14,800 rpm for 20 min, the supernatant was removed, 500 $\mu$L of reaction solution Buffer B was added, and the mixture was redissolved and washed once.
(2) After washing, the mixture was centrifuged at 14,800 rpm for 20 min, the supernatant was removed, and 500 $\mu$L of reaction solution Buffer B was added, and the mixture was redissolved.

4) Conjugation of antibodies
Antibodies were added to the redissolved microspheres, and the mixture was rotated, mixed, and reacted for 2 h. The different antibody labeling concentrations ranged from 0.02-0.1 mg/mg, that is, 0.02-0.1 mg of antibodies were added per 1 mg of microspheres.

$$\text{Antibody volume} = \text{antibody labeling concentration} \times \text{microsphere mass/antibody concentration.}$$

5) Blocking
After 2.0 h of the coupling reaction, the mixture was centrifuged at 14,800 rpm for 20 min, the supernatant was removed, 500 $\mu$L of blocking solution Buffer C was added, redissolved, and incubated on a rotary incubator for 60 min to perform rotational mixing and blocking.
6) Washing and storing
After blocking, the mixture was centrifuged at 14,800 rpm for 20 min, the supernatant was removed, 250 $\mu$L of storage solution Buffer D (with a final microsphere concentration of 2 mg/mL) was added and redissolved. After resuspension, it was stored at 2-8°C for later use.

**(c) Preparation of labeling zone:**

The fluorescent material-labeled goat anti-human IgG, the fluorescent material-labeled denatured human IgG, and the fluorescent material-labeled goat anti-rabbit IgG obtained in the above steps were coated onto the sample loading zone in a ratio of 20:10:1, and then dried at 37°C for 24 h.

**(d) Preparation of sample zone:**

The sample zone was composed of glass fibers and subjected to a spraying treatment with a solution containing buffer, anti-red blood cell antibody, surfactant, sugar, and other components, which functions to separate interfering substances, such as red blood cells, from the buffer.

**(e) Preparation of detection zone:**

Detection zone (4): the detection zone was a nitrocellulose membrane, which functions as a carrier for a chromatographic reaction, with a first test line, a second test line, a third test line, and a control line sequentially coated thereon.

1) First test line (5, 6, or 7): the first test line was coated on the NC membrane and contained recombinant MCV protein (as set forth in SEQ ID NO: 1), which functions to detect anti-MCV antibodies. The recombinant MCV was diluted to a concentration of 1 mg/ml and streaked onto the first test line on the detection zone at 1.0 $\mu$L/cm.

2) Second test line (5, 6, or 7), the second test line was coated on the NC membrane and contained recombinant CCP protein (as set forth in SEQ ID NO: 2), which functions to detect anti-CCP antibodies. The recombinant CCP was streaked onto the second sub-test line on the detection zone.

3) Third test line (5, 6, or 7): the third test line was coated on NC membrane and contained denatured human IgG), which functions to detect RF. Denatured human IgG was diluted to a concentration of 1 mg/ml and streaked onto the third sub-test line on the detection zone at 1.0 $\mu$L/cm.

4) Control line (8): the control line was coated on the NC membrane and contained rabbit IgG, which functions to indicate the effectiveness of the product. Rabbit IgG at a concentration of 1 mg/mL was streaked onto the control line on the detection zone at 1 $\mu$L/cm.

The coated detection zone (nitrocellulose membrane) mentioned above was dried at 37°C for 24 h. After drying, the nitrocellulose membrane was removed for sealed drying and storage;

**(f) Absorbent pad:**

The absorbent pad is composed of plant fibers, cotton lint, or the like, which functions to absorb excess liquid and provide power for the chromatographic reaction.

**(g) Assembly:**

The chromatographic film (2) obtained in step (d) was attached to the substrate (8), and then the sample loading zone (1) was attached to the end of the chromatographic film (2) near the first test line (3). The absorbent paper (7) was attached to the end of the chromatographic film (2) near the control line (6). The adhered substrate (8) was cut into test strips with a length of 3.5 mm, placed into the plastic case, pressed, and assembled into the kit in an environment where the humidity is less than 30%.

**Example 4: Detection of rheumatoid arthritis using quantum dot immunochromatographic test strips.**

Fluorescent immunochromatographic test strips were prepared according to the procedure shown in Example 3, and rheumatoid arthritis was detected as follows.

1. 10 $\mu$L of blood sample taken from a human was added to a sample diluent to mix homogeneously. 70 $\mu$L of the mixture was added to the sample loading zone of a fluorescent immunochromatographic test strip and left to stand for 15 minutes;

2. Read the results using a quantum dot immunoanalyzer;

If the first sub-detection zone, the second sub-detection zone, and the third sub-detection zone show fluorescently positive results, it is estimated that the donor providing the blood sample has a 99.2% probability of suffering from rheumatoid arthritis;

If the first sub-detection zone and the second sub-detection zone show fluorescently positive results, it is estimated that the donor providing the blood sample has a 94.6% probability of suffering from rheumatoid arthritis;

If the first sub-detection zone and the third sub-detection zone show fluorescently positive results, it is estimated that the donor providing the blood sample has a 91.7% probability of suffering from rheumatoid arthritis;

If the second sub-detection zone and the third sub-detection zone show fluorescently positive results, it is estimated that the donor providing the blood sample has a 93.6% probability of suffering from rheumatoid arthritis;

If the first sub-detection zone shows a fluorescence positive result (or a value greater than 20 U/mL), it is estimated that the donor providing the blood sample has an 88.5% probability of suffering from rheumatoid arthritis;

If the second sub-detection zone shows a fluorescence positive result (or a value greater than 25 U/mL), it is estimated that the donor providing the blood sample has a 92.3% probability of suffering from rheumatoid arthritis;

If the third sub-detection zone shows a fluorescence positive result (or a value greater than 20 IU/mL), it is estimated that the donor providing the blood sample has a 78.8% probability of suffering from rheumatoid arthritis;

According to the X-ray staging criteria, RA patients are divided into a non-bone erosion group (stage I) and a bone erosion group (stages II-IV).

Stage I: joint non-destructive changes or osteoporosis at the joint ends.

Stage II: osteoporosis at the joint ends, with mild subarticular cystic destruction or bone erosion changes; limited joint movement without deformities; muscular atrophy around adjacent joints; extraarticular soft tissue lesions.

Stage III: significant osteoporosis with articular cartilage or bone destruction; joint space narrowing, joint subluxation, etc.; widespread muscular atrophy; extraarticular soft tissue lesions.

Stage IV: fibrous or bony ankylosis in addition to the changes seen in stages II and III.

In some embodiments, if the first test line measurement is in the range of 108.1-334.62 U/mL and the second test line measurement is in the range of 54.22-239.08 U/mL, it is estimated that the condition of the donor providing the blood sample is in stage I according to the X-ray staging criteria.

If the first test line measurement is in the range of 209.69-466.65 U/mL and the second test line measurement is in the range of 192.78-449.86 U/mL, it is estimated that the condition of the donor providing the blood sample is at stage II according to the X-ray staging criteria.

If the first test line measurement is in the range of 215.81-480.71U/mL and the second test line measurement is in the range of 262.96-601.94 U/mL, it is estimated that the condition of the donor providing the blood sample is stage III according to the X-ray staging criteria.

If the first test line measurement is in the range of 129.97-333.55 U/mL and the second test line measurement is in the range of 572.11-1111.47 U/mL, it is estimated that the condition of the donor providing the blood sample is in stage IV according to the X-ray staging criteria.

The first test line measurement, the second test line measurement, or the third test line measurement can be calibrated using a calibration curve.

The first test line measurement, the second test line measurement, or the third test line measurement can be a quantitative result calculated from the fluorescence value using a calibration curve.

**Example 5: Preparation of fluorescent immunochromatographic test strips of Anti-MCV antibody, Anti-CCP antibody, and RF (time-resolved fluorescent immunochromatographic test strips).**

Time-resolved fluorescent microspheres are based on the principle that rare earth ions with a long fluorescence half-life are used as fluorescent materials. Since the Stokes shift of this fluorescent material is large (>150 nm) and its fluorescence lifetime is 5-6 orders of magnitude longer than the fluorescence lifetime of the background material, the signal of this fluorescent material can be measured after the fluorescence of the background material has sufficiently decayed, effectively eliminating interference from non-specific fluorescence and resulting in high sensitivity. As a special functional microsphere, each microsphere can encapsulate thousands of fluorescent molecules, greatly improving the fluorescence labeling efficiency and effectively enhancing analytical sensitivity. Meanwhile, the surface of the fluorescent microspheres is modified with a suitable density of carboxyl or other functional groups for covalent coupling with proteins or antibodies, which enhances the stability of the label.

The method for preparing the time-resolved fluorescent immunochromatographic test strips is as follows:

**(a) Reagents**

1) Carboxyl-modified time-resolved fluorescent microspheres
2) Goat anti-human IgG, denatured human IgG, goat anti-rabbit IgG
3) Activators, EDC, Sulfo-NHS
4) Activating solution Buffer A (0.05 M/L of MES, pH 6.0)
5) Reaction solution Buffer B (0.05 M/L of MES, pH 6.5)
6) Blocking solution Buffer C (0.05 M/L of TRIS-HCl + 1% W/V BSA, pH 8.0)
7) Storage solution Buffer D (0.05 M/L of TRIS-HCl + 1% W/V BSA + 10% W/V trehalose, pH 8.0)

**(b) Marking process**

1% time-resolved fluorescent microspheres can be diluted to different concentrations, such as 10-fold, 5-fold, or original concentrations, for labeling, that is, the microsphere concentration during the reaction can be 1 mg/mL, 2 mg/mL, or 10 mg/mL. The sensitivity differences at the three concentrations were not significant, but the original concentration label tends to aggregate easily. If the concentration exceeds 2 mg/mL, the microsphere concentration is high, making it difficult to observe the dispersion during the reaction process. Therefore, a 10-fold dilution is preferred, with a microsphere concentration of 1 mg/mL.

1) Washing microspheres

50 µL of 1% time-resolved fluorescent microspheres was added to a 1.5 mL centrifuge tube, followed by the addition of 450 µL of activating solution Buffer A. The mixture was shaken at 1000 rpm for 1 min, then centrifuged at 14,800 rpm for 30 min. The supernatant was removed, 400 µL of activating solution Buffer A was added, and the mixture was redissolved and washed once.

2) Activated microspheres

10 mg/mL EDC and Sulfo-NHS solutions were prepared using the activating solution Buffer A. For example, 1 mg of EDC was weighed and dissolved in 100 µL of Buffer A. 50 µL of Sulfo-NHS solution and 50 µL of EDC solution were successively added to the washed microspheres, that is, EDC and NHS used at concentrations of 1 mg/mL. The mixture was then placed on a vortex shaker and shaken at 1000 rpm for 30 min for activation.

3) Post-activation washing

After activation, the mixture was washed, and the solution was replaced with the reaction solution Buffer B:

(1) After activation, the mixture was centrifuged at 14,800 rpm for 20 min, the supernatant was removed, 500 µL of reaction solution Buffer B was added, and the mixture was redissolved and washed once.
(2) After washing, the mixture was centrifuged at 14,800 rpm for 20 min, the supernatant was removed, and 500 µL of reaction solution Buffer B was added, and the mixture was redissolved.

4) Conjugation of antibodies

Antibodies were added to the redissolved microspheres, and the mixture was rotated, mixed, and reacted for 2 h. The different antibody labeling concentrations ranged from 0.02-0.1 mg/mg, that is, 0.02-0.1 mg of antibodies were added per 1 mg of microspheres.

$$\text{Antibody volume} = \text{antibody labeling concentration} \times \text{microsphere mass}/\text{antibody concentration}.$$

5) Blocking

After 2.0 h of the coupling reaction, the mixture was centrifuged at 14,800 rpm for 20 min, the supernatant was removed, 500 µL of blocking solution Buffer C was added, redissolved, and incubated on a rotary incubator for 60 min to perform rotational mixing and blocking.

6) Washing and storing

After blocking, the mixture was centrifuged at 14,800 rpm for 20 min, the supernatant was removed, 250 µL of storage solution Buffer D (with a final microsphere concentration of 2 mg/mL) was added and redissolved. After resuspension, it was stored at 2-8°C for later use.

**(c) Preparation of labeling zone:**

The fluorescent material-labeled goat anti-human IgG, the fluorescent material-labeled denatured human IgG, and the fluorescent material-labeled goat anti-rabbit IgG obtained in the above steps were coated onto the sample loading zone in a ratio of 20:10:1, and then dried at 37°C for 24 h.

**(d) Preparation of sample zone:**

The sample zone was composed of glass fibers and subjected to a spraying treatment with a solution containing buffer, anti-red blood cell antibody, surfactant, sugar, and other components, which functions to separate interfering substances, such as red blood cells, from the buffer.

**(e) Preparation of detection zone:**

Detection zone (4): the detection zone was a nitrocellulose membrane, which functions as a carrier for a chromatographic reaction, with a first test line, a second test line, a third test line, and a control line sequentially coated thereon.

1) First test line (5, 6, or 7): the first test line was coated on the NC membrane and contained recombinant MCV protein (as set forth in SEQ ID NO: 1), which functions to detect anti-MCV antibodies. The recombinant MCV was diluted to a concentration of 1 mg/ml and streaked onto the first test line on the detection zone at 1.0 µL/cm.
2) Second test line (5, 6, or 7), the second test line was coated on the NC membrane and contained recombinant CCP protein (as set forth in SEQ ID NO: 2), which functions to detect anti-CCP antibodies. The recombinant CCP was streaked onto the second sub-test line on the detection zone.

3) Third test line (5, 6, or 7): the third test line was coated on NC membrane and contained denatured human IgG), which functions to detect RF. Denatured human IgG was diluted to a concentration of 1 mg/ml and streaked onto the third sub-test line on the detection zone at 1.0 $\mu$L/cm.

4) Control line (8): the control line was coated on the NC membrane and contained rabbit IgG, which functions to indicate the effectiveness of the product. Rabbit IgG at a concentration of 1 mg/mL was streaked onto the control line on the detection zone at 1 $\mu$L/cm.

The coated detection zone (nitrocellulose membrane) mentioned above was dried at 37°C for 24 h. After drying, the nitrocellulose membrane was removed for sealed drying and storage;

**(f) Absorbent pad:**

The absorbent pad is composed of plant fibers, cotton lint, or the like, which functions to absorb excess liquid and provide power for the chromatographic reaction.

**(g) Assembly:**

The chromatographic film (2) obtained in step (d) was attached to the substrate (8), and then the sample loading zone (1) was attached to the end of the chromatographic film (2) near the first test line (3). The absorbent paper (7) was attached to the end of the chromatographic film (2) near the control line (6). The adhered substrate (8) was cut into test strips with a length of 3.5 mm, placed into the plastic case, pressed, and assembled into the kit in an environment where the humidity is less than 30%.

**Example 6: Detection of rheumatoid arthritis using time-resolved fluorescent immunochromatographic test strips.**

Fluorescent immunochromatographic test strips were prepared according to the procedure shown in Example 5, and rheumatoid arthritis was detected as follows.

1. 10 $\mu$L of blood sample taken from a human was added to a sample diluent to mix homogeneously. 70 $\mu$L of the mixture was added to the sample loading zone of a fluorescent immunochromatographic test strip and left to stand for 15 minutes;

2. Read the results using a time-resolved fluorescence immunoanalyzer;

If the first sub-detection zone, the second sub-detection zone, and the third sub-detection zone show fluorescently positive results, it is estimated that the donor providing the blood sample has a 99.2% probability of suffering from rheumatoid arthritis;

If the first sub-detection zone and the second sub-detection zone show fluorescently positive results, it is estimated that the donor providing the blood sample has a 94.6% probability of suffering from rheumatoid arthritis;

If the first sub-detection zone and the third sub-detection zone show fluorescently positive results, it is estimated that the donor providing the blood sample has a 91.7% probability of suffering from rheumatoid arthritis;

If the second sub-detection zone and the third sub-detection zone show fluorescently positive results, it is estimated that the donor providing the blood sample has a 93.6% probability of suffering from rheumatoid arthritis;

If the first sub-detection zone shows a fluorescence positive result (or a value greater than 20 U/mL), it is estimated that the donor providing the blood sample has an 88.5% probability of suffering from rheumatoid arthritis;

If the second sub-detection zone shows a fluorescence positive result (or a value greater than 25 U/mL), it is estimated that the donor providing the blood sample has a 92.3% probability of suffering from rheumatoid arthritis;

If the third sub-detection zone shows a fluorescence positive result (or a value greater than 20 IU/mL), it is estimated that the donor providing the blood sample has a 78.8% probability of suffering from rheumatoid arthritis;

According to the X-ray staging criteria, RA patients are divided into a non-bone erosion group (stage I) and a bone erosion group (stages II-IV).

Stage I: joint non-destructive changes or osteoporosis at the joint ends.

Stage II: osteoporosis at the joint ends, with mild subarticular cystic destruction or bone erosion changes; limited joint movement without deformities; muscular atrophy around adjacent joints; extraarticular soft tissue lesions.

Stage III: significant osteoporosis with articular cartilage or bone destruction; joint space narrowing, joint subluxation, etc.; widespread muscular atrophy; extraarticular soft tissue lesions.

Stage IV: fibrous or bony ankylosis in addition to the changes seen in stages II and III.

In some embodiments, if the first test line measurement is in the range of 108.1-334.62 U/mL and the second test line measurement is in the range of 54.22-239.08 U/mL, it is estimated that the condition of the donor providing the blood sample is in stage I according to the X-ray staging criteria.

If the first test line measurement is in the range of 209.69-466.65 U/mL and the second test line measurement is in the range of 192.78-449.86 U/mL, it is estimated that the condition of the donor providing the blood sample is at stage II according to the X-ray staging criteria.

If the first test line measurement is in the range of 215.81-480.71U/mL and the second test line measurement is in the range of 262.96-601.94 U/mL, it is estimated that the condition of the donor providing the blood sample is stage III according to the X-ray staging criteria.

If the first test line measurement is in the range of 129.97-333.55 U/mL and the second test line measurement is in the range of 572.11-1111.47 U/mL, it is estimated that the condition of the donor providing the blood sample is in stage IV according to the X-ray staging criteria.

The first test line measurement, the second test line measurement, or the third test line measurement can be calibrated using a calibration curve.

The first test line measurement, the second test line measurement, or the third test line measurement can be a quantitative result calculated from the fluorescence value using a calibration curve.

**Example 7: Preparation of fluorescent immunochromatographic test strips of Anti-MCV antibody, Anti-CCP antibody, and RF (aggregation-induced emission immunochromatographic test strips).**

The aggregation-induced emission (AIE) materials exhibit high luminescence efficiency in the aggregated state, and the use of a high-molecular structure with a rigid backbone can effectively restrict the molecular vibrational structure, significantly enhancing the solid-state luminescence efficiency of the prepared microspheres. Moreover, the large Stokes shift of AIE molecules facilitates the efficient separation of the light source from the signal acquisition window, leading to a significant enhancement in the sensitivity of its fluorescence signal output. This technique utilizes high-efficiency AIE molecules as signal units, and the AIE fluorescent microspheres are polymerized in situ through monomer selection and process optimization. The microspheres' particle size can be effectively controlled within the range of 50 nm-1000 nm, as well as the micron scale (with a particle size CV value of less than 5%), and the microsphere surface can be quantitatively modified as needed (with functional groups such as carboxyl, amino, hydroxyl, streptavidin, biomacromolecules, etc.). Using coupling technology, various antibodies and antigenic proteins can be further linked, expanding their application in immunoassays. Fluorescent microspheres prepared with this technique outperform similar products in terms of light color, efficiency, and stability, achieving full coverage of the emission wavelengths (blue, green, red, etc.). The functional modification of AIE fluorescent microspheres is highly tunable and offers significant advantages in lateral chromatography, cell imaging, microfluidic technology, and fluorescence enzyme-linked immunosorbent assays.

The method for preparing the aggregation-induced emission immunochromatographic test strips is as follows:

**(a) Reagents**

1) Carboxyl-modified aggregation-induced emission microspheres
2) Goat anti-human IgG, denatured human IgG, goat anti-rabbit IgG
3) Activators, EDC, Sulfo-NHS
4) Activating solution Buffer A (0.05 M/L of MES, pH 6.0)
5) Reaction solution Buffer B (0.05 M/L of MES, pH 6.5)
6) Blocking solution Buffer C (0.05 M/L of TRIS-HCl + 1% W/V BSA, pH 8.0)
7) Storage solution Buffer D (0.05 M/L of TRIS-HCl + 1% W/V BSA + 10% W/V trehalose, pH 8.0)

**(b) Marking process**

1% aggregation-induced emission microspheres can be diluted to different concentrations, such as 10-fold, 5-fold, or original concentrations, for labeling, that is, the microsphere concentration during the reaction can be 1 mg/mL, 2 mg/mL, or 10 mg/mL. The sensitivity differences at the three concentrations were not significant, but the original concentration label tends to aggregate easily. If the concentration exceeds 2 mg/mL, the microsphere concentration is high, making it difficult to observe the dispersion during the reaction process. Therefore, a 10-fold dilution is preferred, with a microsphere concentration of 1 mg/mL.

1) Washing microspheres
50 μL of 1% aggregation-induced emission microspheres was added to a 1.5 mL centrifuge tube, followed by the addition of 450 μL of activating solution Buffer A. The mixture was shaken at 1000 rpm for 1 min, then centrifuged at 14,800 rpm for 30 min. The supernatant was removed, 400 μL of activating solution Buffer A was added, and the mixture was redissolved and washed once.
2) Activated microspheres
10 mg/mL EDC and Sulfo-NHS solutions were prepared using the activating solution Buffer A. For example, 1 mg of EDC was weighed and dissolved in 100 $\mu$L of Buffer A. 50 $\mu$L of Sulfo-NHS solution and 50 $\mu$L of EDC solution were successively added to the washed microspheres, that is, EDC and NHS used at concentrations of 1 mg/mL. The mixture was then placed on a vortex shaker and shaken at 1000 rpm for 30 min for activation.

3) Post-activation washing

After activation, the mixture was washed, and the solution was replaced with the reaction solution Buffer B:

(1) After activation, the mixture was centrifuged at 14,800 rpm for 20 min, the supernatant was removed, 500 $\mu$L of reaction solution Buffer B was added, and the mixture was redissolved and washed once.

(2) After washing, the mixture was centrifuged at 14,800 rpm for 20 min, the supernatant was removed, and 500 $\mu$L of reaction solution Buffer B was added, and the mixture was redissolved.

4) Conjugation of antibodies

Antibodies were added to the redissolved microspheres, and the mixture was rotated, mixed, and reacted for 2 h. The different antibody labeling concentrations ranged from 0.02-0.1 mg/mg, that is, 0.02-0.1 mg of antibodies were added per 1 mg of microspheres.

$$\text{Antibody volume} = \text{antibody labeling concentration} \times \text{microsphere mass/antibody concentration.}$$

5) Blocking

After 2.0 h of the coupling reaction, the mixture was centrifuged at 14,800 rpm for 20 min, the supernatant was removed, 500 $\mu$L of blocking solution Buffer C was added, redissolved, and incubated on a rotary incubator for 60 min to perform rotational mixing and blocking.

6) Washing and storing

After blocking, the mixture was centrifuged at 14,800 rpm for 20 min, the supernatant was removed, 250 $\mu$L of storage solution Buffer D (with a final microsphere concentration of 2 mg/mL) was added and redissolved. After resuspension, it was stored at 2-8°C for later use.

**(c) Preparation of labeling zone:**

The fluorescent material-labeled goat anti-human IgG, the fluorescent material-labeled denatured human IgG, and the fluorescent material-labeled goat anti-rabbit IgG obtained in the above steps were coated onto the sample loading zone in a ratio of 20:10:1, and then dried at 37°C for 24 h.

**(d) Preparation of sample zone:**

The sample zone was composed of glass fibers and subjected to a spraying treatment with a solution containing buffer, anti-red blood cell antibody, surfactant, sugar, and other components, which functions to separate interfering substances, such as red blood cells, from the buffer.

**(e) Preparation of detection zone:**

Detection zone (4): the detection zone was a nitrocellulose membrane, which functions as a carrier for a chromatographic reaction, with a first test line, a second test line, a third test line, and a control line sequentially coated thereon.

1) First test line (5, 6, or 7): the first test line was coated on the NC membrane and contained recombinant MCV protein (as set forth in SEQ ID NO: 1), which functions to detect anti-MCV antibodies. The recombinant MCV was diluted to a concentration of 1 mg/ml and streaked onto the first test line on the detection zone at 1.0 $\mu$L/cm.

2) Second test line (5, 6, or 7), the second test line was coated on the NC membrane and contained recombinant CCP protein (as set forth in SEQ ID NO: 2), which functions to detect anti-CCP antibodies. The recombinant CCP was streaked onto the second sub-test line on the detection zone.

3) Third test line (5, 6, or 7): the third test line was coated on NC membrane and contained denatured human IgG), which functions to detect RF. Denatured human IgG was diluted to a concentration of 1 mg/ml and streaked onto the third sub-test line on the detection zone at 1.0 $\mu$L/cm.

4) Control line (8): the control line was coated on the NC membrane and contained rabbit IgG, which functions to indicate the effectiveness of the product. Rabbit IgG at a concentration of 1 mg/mL was streaked onto the control line on the detection zone at 1 $\mu$L/cm.

The coated detection zone (nitrocellulose membrane) mentioned above was dried at 37°C for 24 h. After drying, the nitrocellulose membrane was removed for sealed drying and storage;

**(f) Absorbent pad:**

The absorbent pad is composed of plant fibers, cotton lint, or the like, which functions to absorb excess liquid and provide power for the chromatographic reaction.

**(g) Assembly:**

The chromatographic film (2) obtained in step (d) was attached to the substrate (8), and then the sample loading zone (1) was attached to the end of the chromatographic film (2) near the first test line (3). The absorbent paper (7) was attached to the end of the chromatographic film (2) near the control line (6). The adhered substrate (8) was cut into test strips with a length of 3.5 mm, placed into the plastic case, pressed, and assembled into the kit in an environment where the humidity is less than 30%.

**Example 8: Detection of rheumatoid arthritis using aggregation-induced emission immunochromatographic test strips**

Fluorescent immunochromatographic test strips were prepared according to the procedure shown in Example 7, and rheumatoid arthritis was detected as follows.

1. 10 $\mu$L of blood sample taken from a human was added to a sample diluent to mix homogeneously. 70 $\mu$L of the mixture was added to the sample loading zone of a fluorescent immunochromatographic test strip and left to stand for 15 minutes;

2. Read the results using an aggregation-induced emission immunoanalyzer;

If the first sub-detection zone, the second sub-detection zone, and the third sub-detection zone show fluorescently positive results, it is estimated that the donor providing the blood sample has a 99.2% probability of suffering from rheumatoid arthritis;

If the first sub-detection zone and the second sub-detection zone show fluorescently positive results, it is estimated that the donor providing the blood sample has a 94.6% probability of suffering from rheumatoid arthritis;

If the first sub-detection zone and the third sub-detection zone show fluorescently positive results, it is estimated that the donor providing the blood sample has a 91.7% probability of suffering from rheumatoid arthritis;

If the second sub-detection zone and the third sub-detection zone show fluorescently positive results, it is estimated that the donor providing the blood sample has a 93.6% probability of suffering from rheumatoid arthritis;

If the first sub-detection zone shows a fluorescence positive result (or a value greater than 20 U/mL), it is estimated that the donor providing the blood sample has an 88.5% probability of suffering from rheumatoid arthritis;

If the second sub-detection zone shows a fluorescence positive result (or a value greater than 25 U/mL), it is estimated that the donor providing the blood sample has a 92.3% probability of suffering from rheumatoid arthritis;

If the third sub-detection zone shows a fluorescence positive result (or a value greater than 20 IU/mL), it is estimated that the donor providing the blood sample has a 78.8% probability of suffering from rheumatoid arthritis;

According to the X-ray staging criteria, RA patients are divided into a non-bone erosion group (stage I) and a bone erosion group (stages II-IV).

Stage I: joint non-destructive changes or osteoporosis at the joint ends.

Stage II: osteoporosis at the joint ends, with mild subarticular cystic destruction or bone erosion changes; limited joint movement without deformities; muscular atrophy around adjacent joints; extraarticular soft tissue lesions.

Stage III: significant osteoporosis with articular cartilage or bone destruction; joint space narrowing, joint subluxation, etc.; widespread muscular atrophy; extraarticular soft tissue lesions.

Stage IV: fibrous or bony ankylosis in addition to the changes seen in stages II and III.

In some embodiments, if the first test line measurement is in the range of 108.1-334.62 U/mL and the second test line measurement is in the range of 54.22-239.08 U/mL, it is estimated that the condition of the donor providing the blood sample is in stage I according to the X-ray staging criteria.

If the first test line measurement is in the range of 209.69-466.65 U/mL and the second test line measurement is in the range of 192.78-449.86 U/mL, it is estimated that the condition of the donor providing the blood sample is at stage II according to the X-ray staging criteria.

If the first test line measurement is in the range of 215.81-480.71U/mL and the second test line measurement is in the range of 262.96-601.94 U/mL, it is estimated that the condition of the donor providing the blood sample is stage III according to the X-ray staging criteria.

If the first test line measurement is in the range of 129.97-333.55 U/mL and the second test line measurement is in the range of 572.11-1111.47 U/mL, it is estimated that the condition of the donor providing the blood sample is in stage IV according to the X-ray staging criteria.

The first test line measurement, the second test line measurement, or the third test line measurement can be calibrated using a calibration curve.

The first test line measurement, the second test line measurement, or the third test line measurement can be a quantitative result calculated from the fluorescence value using a calibration curve.

**Example 9:** Precision testing

The T/C value represents the ratio of the signal value measured at the test line (T-line) to the signal value measured at the control line (C-line). This calculation method can effectively improve the precision of the detection results. For example, when the signal value of the test line T is counted separately, the coefficient of variation is 10.81%, and when calculated using T/C, the coefficient of variation decreases to 4.11%. The lower the coefficient of variation, the better the precision of the test results, and the more reliable the reagent.

Precision testing (with a coating concentration of 1 mg/mL) was performed using the time-resolved fluorescent immunochromatographic test strip prepared in Example 5, and the results are shown in Table 1.

Table 1: Calculation of the coefficient of variation CV (CV = SD/AV) using T/C values

| MCV reference (10 U/mL) | T signal value | C signal value | T/C value |
| --- | --- | --- | --- |
| Duplicate test 1 | 4940 | 7944 | 0.621853 |
| Duplicate test 2 | 5125 | 7823 | 0.65512 |
| Duplicate test 3 | 5579 | 8534 | 0.653738 |
| Duplicate test 4 | 5085 | 8356 | 0.608545 |
| Duplicate test 5 | 5283 | 8954 | 0.590016 |
| Duplicate test 6 | 6531 | 10015 | 0.652122 |
| Duplicate test 7 | 5505 | 8872 | 0.620491 |
| Duplicate test 8 | 6636 | 9941 | 0.667538 |
| Duplicate test 9 | 5063 | 7961 | 0.635975 |
| Duplicate test 10 | 5583 | 9255 | 0.603241 |
| Average value (AV) | 5533 | 8765.5 | 0.630864 |
| Standard deviation (SD) | 598.1666 | 793.1097 | 0.0259 |
| Coefficient of variation (CV) (%) | 10.81 | 9.05 | 4.11 |

The fluorescent immunochromatographic test strip for quantitative detection of mutant citrullinated vimentin antibody (Anti-MCV), anti-cyclic citrullinated peptide antibody (Anti-CCP), and rheumatoid factor (RF) of the present application were provided with test lines for Anti-MCV, Anti-CCP, and RF, so that three indicators (Anti-MCV, Anti-CCP, and RF) in human whole blood, plasma, and serum can be simultaneously detected by a single sample addition. The operation is simple, and the cost of reagent and consumables can be reduced. At the same time, it can compensate for the deficiency of a single indicator in diagnosing RA and help improve the accuracy of diagnosing RA, reducing misdiagnosis and missed diagnosis.

**Example 10:** Comparison of fluorescence immunochromatography and colloidal gold method

Since the colloidal gold method performs grayscale analysis, and the fluorescent immunochromatography performs fluorescence intensity analysis; therefore, the sensitivity and accuracy of colloidal gold in quantitative analysis may be inferior to those of fluorescence immunochromatography.

The time-resolved fluorescent immunochromatographic test strips prepared in Example 5 were compared with colloidal gold test strips under different conditions with coating concentrations of 1 or 2 mg/mL, and the experimental data are shown in Table 2 and FIG. 3.

The T/C value means the ratio of the signal value of the instrument reading test line (T-line) to the signal value of the control line (C-line).

Table 2: Comparison of fluorescence immunochromatography and colloidal gold method

| Calibrator concentration (U/mL) | Colloidal gold T/C value (the coating concentration is 1 mg/mL) | Colloidal gold T/C value (the coating concentration is 2 mg/mL) | Fluorescence chromatography T/C value (the coating concentration is 1 mg/mL) | Fluorescence chromatography T/C value (the coating concentration is 2 mg/mL) |
|---|---|---|---|---|
| 0 | 0.35 | 1.12 | 0.03 | 1.03 |
| 10 | 0.41 | 1.46 | 0.64 | 1.6 |
| 25 | 0.58 | 1.89 | 1.53 | 2.27 |
| 50 | 0.91 | 2.5 | 2.72 | 3.13 |
| 100 | 1.51 | 3.29 | 4.32 | 4.53 |
| 250 | 3.11 | 5.06 | 7.90 | 8.23 |
| 500 | 4.63 | 6.47 | 12.19 | 13.03 |
| 1000 | 5.79 | 8.03 | 17.61 | 19.7 |
| 2000 | 6.98 | 9.34 | 24.23 | 28.01 |

**Claims**

1. A fluorescent immunochromatographic test strip for detecting rheumatoid arthritis, comprising a sample loading zone, a detection zone, and an absorbent zone, wherein:

   the detection zone comprises a first sub-detection zone coated with a mutant citrullinated vimentin (MCV), a second sub-detection zone coated with a cyclic citrullinated peptide (CCP), and a third sub-detection zone coated with a denatured human IgG;
   the coating amount of the mutant citrullinated vimentin, the cyclic citrullinated peptide, or the denatured human IgG is independently 0.15-0.75 $\mu$g;
   the amino acid sequence of the mutant citrullinated vimentin is set forth in SEQ ID NO: 1;
   the amino acid sequence of the cyclic citrullinated peptide is set forth in SEQ ID NO: 2;
   the sample loading zone contains fluorescent material-labeled goat anti-human IgG, fluorescent material-labeled denatured human IgG, and fluorescent material-labeled goat anti-rabbit IgG, in a mass ratio of 20:10:1.

2. The fluorescent immunochromatographic test strip of claim 1, further comprising a substrate.

3. The fluorescent immunochromatographic test strip of claim 1, wherein the sample loading zone comprises a sample zone for receiving a sample, and a labeling zone having the fluorescent material-labeled goat anti-human IgG, the fluorescent material-labeled denatured human IgG, and the fluorescent material-labeled goat anti-rabbit IgG coated thereon.

4. The fluorescent immunochromatographic test strip of claim 1, wherein the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

5. The fluorescent immunochromatographic test strip of claim 1, further comprising a quality control zone located downstream of the first sub-detection zone, the second sub-detection zone, and the third sub-detection zone in the flow direction of the sample on the test strip.

6. The fluorescent immunochromatographic test strip of claim 5, wherein the quality control zone is coated with rabbit IgG, with a coating amount of 0.15-0.75 $\mu$g.

7. The fluorescent immunochromatographic test strip of claim 1, wherein:
   the sample loading zone, the detection zone, and the absorbent zone are arranged sequentially from upstream to downstream in the flow direction of a sample on the test strip.

8. The fluorescent immunochromatographic test strip of claim 1, wherein the coating concentration of the mutant citrullinated vimentin, the cyclic citrullinated peptide, or the denatured human IgG is independently 0.5-1.5 mg/mL.

9. A kit comprising the fluorescent immunochromatographic test strip of any one of claims 1 to 8 and instructions for use.

10. Use of the fluorescent immunochromatographic test strip of any one of claims 1 to 8 in the preparation of a kit for the detection of rheumatoid arthritis.

11. A method for assessing whether a subject suffers from rheumatoid arthritis or determining the severity of rheumatoid arthritis in a subject, comprising the following steps:

a. loading a blood sample from the subject to a sample loading zone of a fluorescent immunochromatographic test strip, wherein:

the fluorescent immunochromatographic test strip comprises the sample loading zone, a detection zone, and an absorbent zone,
the detection zone comprises a first sub-detection zone coated with mutant citrullinated vimentin, a second sub-detection zone coated with cyclic citrullinated peptide, and a third sub-detection zone coated with denatured human IgG,
the coating amount of the mutant citrullinated vimentin, the cyclic citrullinated peptide, or the denatured human IgG is independently 0.15-0.75 $\mu$g,
the amino acid sequence of the mutant citrullinated vimentin is set forth in SEQ ID NO: 1,
the amino acid sequence of the cyclic citrullinated peptide is set forth in SEQ ID NO: 2, and
the sample loading zone contains fluorescent material-labeled goat anti-human IgG, fluorescent material-labeled denatured human IgG, and fluorescent material-labeled goat anti-rabbit IgG, in a mass ratio of 20:10:1; and

b. determining qualitative or quantitative results generated from the fluorescent materials in the first sub-detection zone, the second sub-detection zone, and the third sub-detection zone, and assessing whether the subject suffers from rheumatoid arthritis or determining the severity of rheumatoid arthritis based on the qualitative or quantitative results.

12. The method of claim 11, wherein the blood sample is a whole blood sample, a plasma sample, or a serum sample.

13. The method of claim 11, wherein the fluorescent immunochromatographic test strip further comprises a substrate.

14. The method of claim 11, wherein the sample loading zone comprises a sample zone for receiving the sample, and a labeling zone having the fluorescent material-labeled goat anti-human IgG, the fluorescent material-labeled denatured human IgG, and the fluorescent material-labeled goat anti-rabbit IgG coated thereon.

15. The method of claim 11, wherein the fluorescent material is selected from the group consisting of fluorescein isothiocyanate or a derivative thereof, rhodamine or a derivative thereof, phycoerythrin, a fluorescent polystyrene microsphere, a time-resolved microsphere, an upconversion luminescent microparticle, a quantum dot microsphere, and an aggregation-induced emission microsphere.

16. The method of claim 11, wherein the fluorescent immunochromatographic test strip further comprises a quality control zone located downstream of the first sub-detection zone, the second sub-detection zone, and the third sub-detection zone in the flow direction of the sample on the test strip.

17. The method of claim 16, wherein the quality control zone is coated with a rabbit IgG, with a coating amount of 0.15-0.75 $\mu$g.

18. The method of claim 11, wherein:
the sample loading zone, the detection zone, and the absorbent zone are arranged from the upstream to the downstream of the flow direction of the sample on the test strip.

**19.** The method of claim 11, wherein the coating concentration of the mutant citrullinated vimentin, the cyclic citrullinated peptide, or the denatured human IgG is independently 0.5-1.5 mg/mL.

Fig. 1

Fig. 2

Fig. 3

## INTERNATIONAL SEARCH REPORT

| International application No. |
|---|
| **PCT/CN2024/104996** |

| A. | CLASSIFICATION OF SUBJECT MATTER |
|---|---|

G01N 33/564(2006.01)i; G01N 33/543(2006.01)i; G01N 33/68(2006.01)i; G01N 33/58(2006.01)i

According to International Patent Classification (IPC) or to both national classification and IPC

| B. | FIELDS SEARCHED |
|---|---|

Minimum documentation searched (classification system followed by classification symbols)

IPC:G01N

Documentation searched other than minimum documentation to the extent that such documents are included in the fields searched

Electronic data base consulted during the international search (name of data base and, where practicable, search terms used)

CNTXT, ENTXTC, CNKI, web of science: 睿盟创新, 林潮喜, 陈棠明, 类风湿性关节炎, 类风湿关节炎, 突变型瓜氨酸, 环瓜氨酸, 类风湿因子, 荧光, 免疫层析, 羊抗人, 羊抗兔, rheumatoid arthritis, RA, cyclic citrullinated peptides, mutant citrullinated vimentin, MCV, CCP, electrochemiluminescence, ECL, fluorescence, immunoassay, FIA, IgG, RF

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 117110603 A (SHENZHEN RUIMENG INNOVATION BIOTECHNOLOGY CO., LTD.) 24 November 2023 (2023-11-24) description, paragraphs [0006]-[0036], and figures 1-3 | 1-10 |
| Y | CN 108956982 A (GUANGZHOU HUAAO BIO-TECH CO., LTD.) 07 December 2018 (2018-12-07) claim 1, and description, paragraphs [0007]-[0033], and figures 1-7 | 1-10 |
| Y | CN 111308096 A (SHENZHEN RUIMENG INNOVATION BIOTECHNOLOGY CO., LTD.) 19 June 2020 (2020-06-19) description, paragraphs [0006]-[0040], and figures 1 and 2 | 1-10 |
| A | CN 113049814 A (SECOND AFFILIATED HOSPITAL OF NANCHANG UNIVERSITY) 29 June 2021 (2021-06-29) entire document | 1-10 |
| A | CN 203838159 U (ZHENGZHOU LANGFENG BIOTECHNOLOGY CO., LTD. et al.) 17 September 2014 (2014-09-17) entire document | 1-10 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 August 2024** | **13 August 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/104996**

### C. DOCUMENTS CONSIDERED TO BE RELEVANT

| Category* | Citation of document, with indication, where appropriate, of the relevant passages | Relevant to claim No. |
|---|---|---|
| A | CN 104459140 A (CHONGQING QIANDE BIOTECHNOLOGY CO., LTD.) 25 March 2015 (2015-03-25) <br> entire document | 1-10 |
| A | CN 203732542 U (SHANGHAI KEXIN BIOTECH CO., LTD.) 23 July 2014 (2014-07-23) <br> entire document | 1-10 |
| A | US 2015080245 A1 (CATHOLIC UNIVERSITY INDUSTRY-ACADEMIC COOPERATION FOUNDATION) 19 March 2015 (2015-03-19) <br> entire document | 1-10 |
| A | 陈黎 等 (CHEN, Li et al.). "四项指标联合检测在类风湿关节炎诊断中的价值 (Diagnostic Value of Four Auto-antibodies in Patients with Rheumatoid Arthritis)" <br> 湖北医药学院学报 (Journal of Hubei University of Medicine), <br> Vol. 30, No. (1), 25 February 2011 (2011-02-25), <br> ISSN: 2096-708X, <br> pp. 65-67 | 1-10 |

Form PCT/ISA/210 (second sheet) (July 2022)

| INTERNATIONAL SEARCH REPORT | International application No. |
|---|---|
| | **PCT/CN2024/104996** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

         ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
|---|
| **PCT/CN2024/104996** |

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **11-19**
   because they relate to subject matter not required to be searched by this Authority, namely:

   Claims 11-19 relate to a method for evaluating whether a subject has rheumatoid arthritis or evaluating a degree of illness, which belongs to a diagnostic method. Therefore, claims 11-19 belong to subject matter for which no international search is required as defined in PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

# EP 4 745 574 A1

## INTERNATIONAL SEARCH REPORT
### Information on patent family members

International application No.

**PCT/CN2024/104996**

| Patent document cited in search report | | | Publication date (day/month/year) | Patent family member(s) | | | Publication date (day/month/year) |
|---|---|---|---|---|---|---|---|
| CN | 117110603 | A | 24 November 2023 | None | | | |
| CN | 108956982 | A | 07 December 2018 | CN | 108956982 | B | 20 May 2022 |
| CN | 111308096 | A | 19 June 2020 | None | | | |
| CN | 113049814 | A | 29 June 2021 | None | | | |
| CN | 203838159 | U | 17 September 2014 | None | | | |
| CN | 104459140 | A | 25 March 2015 | None | | | |
| CN | 203732542 | U | 23 July 2014 | None | | | |
| US | 2015080245 | A1 | 19 March 2015 | KR | 20130083810 | A | 23 July 2013 |
| | | | | KR | 101458100 | B1 | 05 November 2014 |
| | | | | US | 9347941 | B2 | 24 May 2016 |
| | | | | WO | 2013105721 | A1 | 18 July 2013 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310870599